(19) **Europäisches Patentamt / European Patent Office / Office européen des brevets**

(11) **EP 4 752 531 A1**

(12) **EUROPEAN PATENT APPLICATION**
published in accordance with Art. 153(4) EPC

(43) Date of publication:
**03.06.2026 Bulletin 2026/23**

(21) Application number: **24849191.2**

(22) Date of filing: **30.07.2024**

(51) International Patent Classification (IPC):
**G01N 21/27** [(2006.01)]    **G01N 33/48** [(2006.01)]
**G01N 33/483** [(2006.01)]

(52) Cooperative Patent Classification (CPC):
**G01N 21/31; G01N 15/01; G01N 21/27;**
**G01N 21/51; G01N 33/48; G01N 33/483;**
G01N 2201/1296

(86) International application number:
**PCT/JP2024/027247**

(87) International publication number:
**WO 2025/028546 (06.02.2025 Gazette 2025/06)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **01.08.2023 JP 2023125651**

(71) Applicants:
• **National University Corporation Nara Institute of Science and Technology**
  **Ikoma-shi, Nara 630-0192 (JP)**
• **Kinki University**
  **Higashi-Osaka-shi**
  **Osaka 577-8502 (JP)**

(72) Inventors:
• **HOSOKAWA Yoichiroh**
  **Ikoma-shi, Nara 630-0192 (JP)**
• **FUJII Mikiya**
  **Ikoma-shi, Nara 630-0192 (JP)**
• **ONISHI Risa**
  **Ikoma-shi, Nara 630-0192 (JP)**
• **TSURI Yuka**
  **Ikoma-shi, Nara 630-0192 (JP)**
• **YASUKUNI Ryohei**
  **Ikoma-shi, Nara 630-0192 (JP)**
• **ITO Akihiko**
  **Osakasayama-shi, Osaka 589-8511 (JP)**
• **WAKASA Tomoko**
  **Ikoma-shi, Nara 630-0293 (JP)**

(74) Representative: **Casalonga**
  **Casalonga & Partners**
  **Bayerstraße 71/73**
  **80335 München (DE)**

(54) **METHOD AND DEVICE FOR DISCRIMINATING CELLS BY ELECTROMAGNETIC WAVE SCATTERING SPECTRUM**

(57)    Provided is a method for using a sample containing cells collected from a patient to discriminate, with high sensitivity and high accuracy, normal cells and abnormal cells contained in the sample. A sample containing cells collected from a patient is irradiated with electromagnetic waves, an electromagnetic wave scattering spectrum from the cells in the sample is measured, and normal cells and abnormal cells are discriminated on the basis of the characteristics of the electromagnetic wave scattering spectrum. The discrimination uses a trained model in which the electromagnetic wave scattering spectra of normal cells and abnormal cells are trained. The sample may be a living cell, or may be a sample that is immobilized by alcohol or formalin and dyed. The electromagnetic wave scattering spectrum may use either or both of a scattering spectrum obtained through Rayleigh scattering and a scattering spectrum obtained through Mie scattering.

EP 4 752 531 A1

[Fig. 12]

**Description**

[Technical Field]

The present invention relates to a method and a device that use a sample containing cells collected from a patient and easily detect denatured abnormal cells from normal cells contained in the sample with high sensitivity and high accuracy. In particular, the present invention relates to a method and a device for discriminating tumor cells, which are difficult to discriminate with an optical microscope.

[Background Art]

One of testing methods for malignant tumors is pathological examination by observing a tissue section of a suspected patient under an optical microscope. In this diagnosis, a clinician diagnoses a suspected malignant tumor, and if it is suspicious, a pathologist receives a pathological specimen such as a tissue specimen or a cytologic specimen from the clinician and diagnoses the type and condition of the tumor. Based on the diagnosis, a treatment plan is formulated. Thus, the pathological diagnosis plays a significantly important role in the medical setting. However, in Japan, compared to other developed countries, the number of pathologists is small, and a small number of pathologists pathologically diagnoses a very large amount of specimens. Furthermore, in pathological diagnosis with an optical microscope, it is often difficult to distinguish between tumor cells and normal cells, and the diagnosis may depend on the experience of the pathologist. Under such circumstances, in recent years, in order to solve these problems, artificial intelligence (AI) analysis based on machine learning of optical microscope observation images of pathological specimens is attracting attention.

In observation of specimens only with an optical microscope, some tumor cells cannot be distinguished from normal cells even by experienced pathologists. Differences between tumor cells and normal cells appear not only in the forms of cells or intracellular organelles of micrometer sizes that can be observed with an optical microscope but also in turbulences in the microstructures of cytoskeletons and intracellular organelles with sizes of micrometer or less (nanosizes), protein denaturation, and so on. It is believed that diagnostic accuracy of pathological specimens can be improved if information on these nanosizes can be detected and analyzed by light scattering spectra in addition to optical microscope images. Furthermore, it is expected that AI analysis at a high speed and with high accuracy is realized if high-density information can be obtained at a higher speed than optical microscope observation.

It is required that a sample be appropriately produced before analysis and a precise testing procedure is adopted. In cytodiagnosis, usually, first of all, a collected sample is smeared on a slide glass and is immediately dehydrated and fixed with ethanol. When the sample is dry, rehydration treatment is performed, and fixation with 95% ethanol is then performed. After fixation, processing such as Papanicolaou staining is carried out. In pathological diagnosis, usually, first of all, a collected sample is fixed with formalin to produce a paraffin-embedded block. Sections are produced from the block and are subjected to hematoxylin eosin (HE) staining or the like. When a tumor is suspected, immunostaining procedures corresponding to various tumor markers are adopted, and highly accurate discrimination is performed by a pathologist. The stained sample is observed, and discrimination is performed based on enlargement of the nucleus size, multinucleation, and also abnormalities in the intranuclear microstructure, which are characteristics of tumor cells. However, there are many types of tumor cells that are significantly difficult to be distinguished based on the difference. For example, in cytodiagnosis of malignant mesothelioma, which is one of rare cancers, in many cases, even specialists have difficulty in discrimination of mesothelioma cells from reactive mesothelial cells and adenocarcinoma cells, so a more appropriate procedure for discrimination is demanded.

Since a pathological specimen-producing process (a series of operational procedures including fixing and staining steps) takes a lot of effort and time, it is desirable to discriminate tumors before fixing and staining during an operation or immediately after collection. As a method, for example, Patent Literature 1 discloses a method for detecting abnormal cells (tumor cells) by irradiating living cells with laser light directly without performing a staining process and so on and spectroscopically analyzing induced emission light emitted from the living cells. Specifically, the aim is to detect abnormal cells by irradiating cells with a visible light laser and spectroscopically analyzing (spectrometry) the induced emission light from the biological sample. In examples of this invention, the scattered light component of laser light irradiated to living cells is cut by a filter, and the light scattering which is an object of the present invention is not an object of the detection information. In the spectrum of induced emission light, since the discrimination accuracy of the condition in a cell is low, some types of tumor cells cannot be detected.

Alternatively, as a method for staining living cells during an operation or immediately after collection without fixing, for example, Non-Patent Literature 1 describes fluorescent staining using 5-aminolevulinic acid (5-ALA). 5-ALA is specifically incorporated into tumor cells and is metabolized (converted) into protoporphyrin IX, which is a fluorescent molecule, and is accumulated in tumor cells. A method for discriminating tumor cells by detecting the fluorescence of the protoporphyrin IX is generally used for treatment of skin cancer in Europe and America, and is used also in Japan as an intraoperative fluorescent diagnostic agent for brain tumors and bladder cancer in order to remove the entire tumor area.

However, in this method that discriminates tumor cells only by the presence or absence of fluorescence from protoporphyrin IX, it is difficult to discriminate tumor cells at higher accuracy than the observation of a pathological specimen stained with a variety of dyes, and the type of tumor cells cannot be distinguished. In addition, the staining method that utilizes the metabolism of 5-ALA by living tumor cells cannot be applied to staining of fixed specimens and therefore cannot be used in a method for easily detecting, with high sensitivity and high accuracy, normal cells and abnormal cells (tumor cells) contained in a pathological specimen including cells collected from a patient, which is an object of the present invention.

Furthermore, a method for discriminating between normal cells and abnormal cells (tumor cells) by irradiating living cells with electromagnetic waves, such as light, directly without subjecting the living cells to a staining process or the like and using a scattered electromagnetic wave spectrum has been investigated using various procedures. However, the methods have not discriminated tumor cells at higher accuracy than observation of a pathological specimen.

For example, Non-Patent Literatures 2 and 3 have reported examples of detecting tumor cells by irradiation with visible light and analysis of scattered light observed as backward scattering light by reflection on the surface of living tissue. Non-Patent Literature 2 shows that enlargement of the nucleus size, which is a feature of tumor cells, can be detected by analyzing the angle dependency of the Mie scattering light intensity that is scattered backward based on the size of the nucleus of a living cell. Non-Patent Literature 3 shows that the presence of an enlarged nucleus in a tumor cell is similarly detected by analyzing the wavelength dependency of the Mie scattering light intensity that is scattered backward from a living cell. As in these examples, changes in the scattering directions or spectral shapes of Mie scattering light and Rayleigh scattering due to the size of nucleus of a cell having a simple shape that is close to a sphere can be predicted theoretically based on physics. However, it is impossible to predict physically and definitely changes in the scattering directions or spectral shapes of Mie scattering and Rayleigh scattering originated from the more complex shape in a cell, and the type of the tumor cell cannot be distinguished.

Patent Literature 2 discloses a method for identifying the types of cells by measuring fluid containing a mixture of various types of cells using a flow cytometer. This method analyzes the time-series signal of an optical image or spectrum obtained while changing the relative position of the illumination pattern and the observed object in a micro flow path by AI based on machine learning not requiring physical and definite prediction. By this method, analysis for distinguishing between normal cells and abnormal cells in a large number of cells is possible using AI based on machine learning. However, the analysis is applicable only when a time-series signal is obtained from cells flowing at a constant flow rate such as with a flow cytometer and is difficult to apply to cells that are bonded and immobilized to a substrate, such as a pathological specimen. In contrast, the present invention is characterized by not requiring a time-series signal. The invention described in Patent Literature 2 cannot be used in a method for easily detecting, with high sensitivity and high accuracy, normal cells and tumor cells contained in an immobilized sample including cells collected from a patient, which is an object of the present invention.

As described above, the investigation for discrimination between normal cells and abnormal cells by various measurement methods using living cells as the objects is mainly aimed at identifying the area of tumor tissue particularly during an operation. However, the above-described methods do not use a more detailed discrimination method using a tumor marker or the like that is expressed in various types of tumors and therefore detect merely the presence of tumor cells or tissue including tumor cells.

It is an object of the present invention to provide a method for discriminating between normal cells and abnormal cells in living cells and also to provide a method for discriminating between normal cells and abnormal cells in a fixed and stained pathological specimen produced according to a process of known pathological diagnosis by measurement of a light scattering spectrum of the pathological specimen based on the morphological characteristics of nanometer to sub-nanometer size, such as the microscopic shape of cells, the shape of nuclei, and intranuclear microstructure, and analysis of the light scattering spectrum using machine learning.

That is, it is an object of the present invention to provide a method for easily detecting, with high sensitivity and high accuracy, normal cells and abnormal cells in a sample containing cells collected from a patient. The sample is a pathological specimen that is collected from a patient and is along the flow of known pathological diagnosis through fixing and staining steps or an object on the stage previous to the fixing and staining steps. The object is to provide means for easily changing an existing diagnosis that depends on the experience and ability of a pathologist to one having high sensitivity and high accuracy without depending on the experience and ability of a pathologist. In particular, the object is to provide a method for discriminating tumor cells that are difficult to be discriminated with an optical microscope.

[Citation List]

[Patent Literature]

[Patent Literature 1] JP 1983-118948 A
[Patent Literature 2] JP 6959614 B

[Non-Patent Literature]

[Non-Patent Literature 1] Shunichiro Ogura, et al., "Dormant cancer cell-targeted photodynamic therapy using 5-aminolevulinic acid", The Journal of Japan Society for Laser Surgery and Medicine, Vol. 43, No. 4, pp. 238-248 (2023).
[Non-Patent Literature 2] A. Wax, et. al., "Cellular organization and substructure measured using angle-resolved low-coherence interferometry", Biophysical journal, 82(4), pp. 2256-2264 (2002).
[Non-Patent Literature 3] V. Backman, et. al., "Polarized light scattering spectroscopy for quantitative measurement of epithelial cellular structures in situ", IEEE Journal of Selected Topics in Quantum Electronics, 5(4), pp. 1019-1026 (1999).

[Summary of Invention]

[Technical Problem]

It is an object to provide a method and a device for detecting, with high sensitivity and high accuracy, discrimination between normal cells and abnormal cells contained in a sample containing cells collected from a patient. In particular, it is an object to provide a method and a device for discriminating tumor cells that are particularly difficult to be discriminated with an optical microscope.

[Solution to Problem]

In order to solve the above problems, in the cell discrimination method of the present invention, a sample containing cells collected from a patient (cell sample) is irradiated with electromagnetic waves to obtain an electromagnetic wave scattering spectrum of the sample, and normal cells and abnormal cells are discriminated based on the information obtained from the spectrum. Scattering of light is influenced by reflection or refraction of a structure having a size approximately equal to or smaller than the wavelength (100 nm to 1 $\mu$m) of the light. Light scattering by structures of approximately the same size as the wavelength (micrometer scale) is referred to as Mie scattering, and light scattering by structures of smaller than the wavelength (submicrometer scale) is referred to as Rayleigh scattering. The present invention is characterized in that information (spectral shapes) included in the optical spectra of Rayleigh scattering and Mie scattering is used in identification of abnormal cells. Optical images also include information by Rayleigh scattering, Mie scattering, and so on but do not include information beyond the diffraction limit. Information on an intracellular structure of a submicron scale that cannot be observed with an optical microscope beyond the diffraction limit can be obtained by analyzing the light scattering spectrum as a feature of the present invention, and it is new in that the information is extracted from the light scattering spectrum by a mathematical method not based on physical and chemical interpretation. When a cell sample is irradiated with light, the scattering direction and spectral shape of light are changed by characteristics such as the microscopic shape of submicrometer-scale cells, the shape of nuclei, and the intranuclear microstructure, in addition to the shapes of micrometer-scale cells and cell nuclei. Furthermore, when cells are subjected to treatment for emphasizing a feature of the structure of abnormal cells, the type of included protein by staining with a dye as in a pathological specimen, the light scattering is strongly influenced by the dye. It is presumed that all of these informations are definitely reflected on the light scattering spectrum of a pathological specimen, therefore, it is too complex to definitely combine the feature of a simple spectral shape such as undulation that is observed in a light scattering spectrum and the cellular and intracellular structures and to theoretically analyze the spectrum based on physics.

In order to solve this problem, the present invention proposes a method for predicting a pathological diagnosis result by analyzing a light scattering spectrum by machine learning. In the machine learning, the feature quantity of a light scattering spectrum is extracted numerically, not based on physical logic, and the feature quantity is analyzed statistically to learn a prediction model (also referred to as a learning model). A procedure for obtaining a prediction whether cells are normal cells or abnormal cells by inputting another light scatting spectrum to this prediction model is an AI analysis. The numerically determined feature quantity does not necessarily accord with the spectral features perceived by humans visually, and the feature quantity is determined by subtle spectral difference that cannot be distinguished visually in some cases. Thus, interpretation that is essentially different from the classic theoretical analysis based on physics is brought.

That is, in the present invention, in the causal relationship between a cell and a microstructure in the cell reflecting the difference between normal cells and abnormal cells, a problem in interpretation of the data on a light scattering spectrum that the causal relationship is too complex to allow theoretical analysis based on physics, has been solved by analysis using machine learning that is a numerical analysis not based on physical logic.

[Effect of Invention]

According to the present invention, an effect of providing a method for detecting discrimination between normal cells and abnormal cells with high sensitivity and high accuracy can be obtained. Tumor cells that are difficult to be discriminated by diagnosis by conventional optical microscope observation by a pathologist can be easily discriminated, with diagnosis not depending on the experience and degree of proficiency of a pathologist, and an increase in the accuracy of the diagnosis can be supported.

[Brief Description of Drawings]

[Fig. 1] Fig. 1 is drawings illustrating an outline of a measurement system: (a) an entire configuration view of the measurement system, (b) an enlarged view of a lighting part for dark-field image observation, and (c) an enlarged view of a lighting part for bright-field image observation.
[Fig. 2] Fig. 2 shows a configuration of an MDCK (Madin-Darby Canine Kidney) cell sample.
[Fig. 3] Fig. 3 shows comparison of a bright-field image and a dark-field image of MDCK cells when Cytochalasin D was not added (a) and was added (b).
[Fig. 4] Fig. 4 shows (a) comparison of normalized light scattering efficiency spectra ($SES_{n710}$) of MDCK cells immersed in a culture medium immediately and 60 minutes after the start of measurement and (b) changes in the normalized light scattering efficiency spectra ($SES_{n710}$) of cells every 10 minutes from immediately after addition of Cytochalasin D to the cells until 60 minutes later.
[Fig. 5] Fig. 5 shows light scattering efficiency spectra (SES) of cells contained in specimens A to F from normal patients.
[Fig. 6] Fig. 6 shows bright-field images and dark-field images of cells contained in the specimens A to F from normal patients: (a) cells of which the SES included a peak at around 700 nm and (b) cells of which the SES was flat.
[Fig. 7] Fig. 7 shows light scattering efficiency spectra (SES) of cells contained in specimens G, H, and I from patients who are suspected to be suffering from mesothelioma.
[Fig. 8] Fig. 8 shows bright-field images and dark-field images of cells contained in the specimens G, H, and I from patients who are suspected to be suffering from mesothelioma.
[Fig. 9] Fig. 9 shows (a) loadings of a 1st principal component and a 2nd principal component obtained by principal component analysis of a light scattering spectrum and (b) a scatter diagram of percentages (scores) of the 1st principal component and the 2nd principal component included in the light scattering spectrum.
[Fig. 10] Fig. 10 shows conceptual drawings of binary classification by (a) a k-nearest neighbor algorithm method, (b) a decision tree method, and (c) a random forest method.
[Fig. 11] Fig. 11 is an explanatory drawing of allocation of learning data and testing data of specimens A to I for verifying the classification accuracy (accuracy rate) of light scattering spectra by a machine learning model.
[Fig. 12] Fig. 12 is a block diagram of a cell discrimination device.
[Fig. 13] Fig. 13 shows normalized light scattering efficiency spectra ($SES_{n710}$) of cells contained in specimens A to I.
[Fig. 14] Fig. 14 shows the contribution rates and loading results of the 1st principal component (PC1) to the $10^{th}$ principal component.
[Fig. 15] Fig. 15 shows scatter diagrams of scores of $n^{th}$ principal component and $m^{th}$ principal component in $SES_{n710}$ of cells.
[Fig. 16] Fig. 16 is an explanatory drawing of the malignancy of tumor cells.
[Fig. 17] Fig. 17 is an explanatory drawing of support of pathological diagnosis.
[Fig. 18] Fig. 18 shows normalized light scattering efficiency spectra ($SES_{n710}$) of concrete two types of living cells.
[Fig. 19] Fig. 19 shows scatter diagrams of scores of $n^{th}$ principal component and $m^{th}$ principal component in $SES_{n710}$ of concrete two types of cells.
[Fig. 20] Fig. 20 shows explanatory drawings of scattering light spectrum-measuring methods.

[Description of Embodiments]

The present invention discriminates unknown cells by obtaining a light scattering spectrum of scattering light generated by irradiating a sample containing cells collected from a patient with electromagnetic waves, numerically extracting a feature quantity included in the light scattering spectrum in order to discriminate between normal cells and abnormal cells based on a difference in characteristics of light scattering spectra of normal cells and abnormal cells, and creating a learning model based on the feature quantity.

A cell includes various cell organelles represented by a cell nucleus in cytosol that is distinguished from the outside by a cell membrane. It is known that characteristic changes occur in the sizes and microstructures of various cell organelles between normal cells and abnormal cells. The present invention intends to capture characteristics of abnormal cells including various tumor cells that play a particularly important role in pathological diagnosis as feature quantity of a light scattering spectrum but is not limited thereto, and provides a method for detecting and analyzing various intracellular and extracellular microstructures by using light scattering spectrometry.

Denaturation of cells and intracellular structure occurs by a variety of causes, and may be induced by aging or a chemical or physical disorder or may be induced by stimulation by various physiologically active substances, infection, canceration of cells, and so on; it is important to detect abnormalities of cells early by capturing a morphological change in the nucleus and other cell organelles included in the cells. As to denaturation of cells, denaturation in the shape and nuclei of cells particularly occupies an important position in various pathological diagnoses. For example, as characteristics of tumor cells, cell aggregate formation, enlargement of the cell nucleus size and multinucleation, enlargement of the nucleolus size, narrowing of the cytosol, and so on may be recognized. In addition, in tumor cells, abnormalities in the polymerization state of cytoskeleton protein such as actin and keratin may occur.

Many malignant tumors become more mobile by canceration, and interstitial invasion and metastasis to another organ across the basement membrane are caused by denaturation of epithelial cells (epithelial-mesenchymal transition). However, it is known that at this time, reorganization of actin filaments that maintain the cytoskeleton occurs and filopodium is formed in the cell membrane to increase the mobility of the cells. Accordingly, denaturation of cells by a tumor or the like can be detected by changes in the cell shape, the shape and size of cell nucleus, intracellular organelles, and cytoskeleton, as a micrometer to submicrometer shape change. The present invention uses a cell discrimination method for discriminating between normal cells and abnormal cells by irradiating a sample containing cells collected from a patient with electromagnetic waves, obtaining an electromagnetic wave scattering spectrum of the cells in the sample, and using a learning model obtained by learning features of the electromagnetic wave scattering spectra of normal cells and abnormal cells. The sample may be living cells or may be a sample fixed and stained with alcohol or formalin.

Conventionally, capturing characteristics of cells by using light scattering has been extensively studied. The light scattering phenomenon is characterized by the particle diameter parameter ($\alpha$; $\alpha = \pi d/\lambda$) represented by the ratio of the diameter (d) of scatterer and the wavelength ($\lambda$) of the electromagnetic wave to be observed and the relative refractive index of the scatterer. When $\alpha \ll 1$, Rayleigh scattering is the main, and the scattered light intensity increases with a decrease in the wavelength. When $\alpha$ is near 1, Mie scattering is the main, and the scattered light intensity is at a constant value without depending on $\alpha$. Furthermore, when $\alpha$ is 10 or more, a diffraction phenomenon due to scattered light interference occurs and exhibits complicated behavior with respect to the scattering angle and $\alpha$.

In the present invention, as to the scattering spectrum, one or both of the scattering spectrum by Rayleigh scattering or the scattering spectrum by Mie scattering can be used. More preferably, as the scattering spectrum, one or both of the forward scattering spectrum by Rayleigh scattering or the forward scattering spectrum by Mie scattering can be used. Furthermore, a difference between the electromagnetic wave scattering spectra of normal cells and abnormal cells is due to an increase or decrease of scattering caused by a change in size of the cell structure which is smaller than or equal to the wavelength of visible light.

The wavelength of the electromagnetic waves that are used for the observation in the present invention is preferably 100 nm to 1 um, and visible light having a wavelength range of 400 to 750 nm is particularly preferable. The electromagnetic waves used are also in an ultraviolet to infrared light range. Furthermore, in the use of the present invention, it is possible to perform a method in which normal cells and abnormal cells are discriminated by using electromagnetic waves in a wavelength range from X-ray to ultraviolet rays to obtain an electromagnetic wave scattering spectrum of an even smaller area of cells in a sample and using a learning model obtained by learning features of the electromagnetic wave scattering spectra of normal cells and abnormal cells.

The present invention is characterized in that structural characteristics of a scatterer are detected with high sensitivity and high accuracy by observing the scattered light from individual cells. In particular, since the structure constituting a cell, such as the nucleus and cytoskeleton, is taken up as the scatterer, the range of $\alpha$ includes a range of 0.1 $< \alpha <$ 150. Since a cell nucleus has a size in a range from several micrometers to about 10 $\mu$m, when the wavelength of the electromagnetic waves to be observed is approximately 500 nm, the value of $\alpha$ is in a range of about 20 to about 120. In this case, the scattered light intensity significantly depends on the scattering angle, and most scattering is observed as forward scattering in the same direction as that of the incident light. When a method of the present invention is used, as observation of the scattered light from cells, the light scattered in any direction relative to the incident angle of electromagnetic waves which is irradiated to the cells is detected to obtain an electromagnetic wave scattering spectrum of the cells in a sample, and a cell discrimination method for discriminating between normal cells and abnormal cells can be performed using a learning model obtained by learning features of the electromagnetic wave scattering spectra of normal cells and abnormal cells.

It is known that when the cross-sectional area of the scatterer is larger than the wavelength of the electromagnetic waves to be observed, the scattering efficiency varies in a complex manner with respect to $\alpha$ and that when the relative refractive index of cellular components in a medium such as water is obtained, the scattering efficiency significantly depends on the size of the scatterer and the wavelength of the electromagnetic waves. Non-Patent Literature 4 (M. Xu et. al., "Unified Mie and fractal scattering by cells and experimental study on application in optical characterization of cellular and subcellular structures", Journal of biomedical optics, 13(2), 024015-024015 (2008)) shows results of comparison of the light scattering spectrum measured by irradiating cells with visible light while changing the scattering angle in a range of 1.1° to 165° to the calculated value. The scattered light from a cell is represented by superposition of a scattered light component from the nucleus in the cell on a scattered light component from the homogeneous cytoplasm and a scattered light component from tiny cell organelles other than the nucleus, and the results of light scattering spectra measured at various scattering angles are described by comparing to calculated values. According to this, in the results of forward scattering at a scattering angle of 1.1°, the scattered light intensity increased with the wavelength, and in the backward scattering, since the phase is inverted, a tendency of a decrease in the scattered light intensity with the wavelength is recognized. It is demonstrated that in contribution of each component of scattered light, the scattering from the cytoplasm of the cell body contributes as the background, and the contribution of the scattering component from the nucleus occupies a large ratio in addition to the fractal "fluctuation" component from the cell organelles. As described above, it is demonstrated that a light scattering spectrum from cells can be precisely measured by irradiating the cells with visible light at a scattering angle in a range of 1.1° to 165°. In the method of the present invention, it is also possible to obtain an electromagnetic wave scattering spectrum from cells in both observation conditions of the forward scattering and the backward scattering and to use a cell discrimination method for discriminating between normal cells and abnormal cells using a learning model obtained by learning the features of the electromagnetic wave scattering spectra of normal cells and abnormal cells.

In Non-Patent Literature 4, the dependency of scattered light intensity on the wavelength becomes more prominent with an increase in the size of the nucleus, and the scattered light intensity significantly increases with the wavelength in forward scattering, but in backward scattering, an inverse tendency is observed. In Non-Patent Literature 4, scattering behavior when cells are denatured by the action of acetic acid has been also investigated, and it is demonstrated that cell organelles denatured by acetic acid are whitened, the scattering intensity significantly increases, and contribution of the "fluctuation" component increases with a decrease in the wavelength.

Thus, in the present invention, analysis is possible by capturing scattering light from any angle of forward scattering and backward scattering. Consequently, it is possible to precisely capture a difference between normal cells and abnormal cells, which is unclear by various conventional procedures.

In Examples of the present invention, a light scattering spectrum obtained by measurement is expressed as two-dimensional data representing a relationship of scattered light intensity with the wavelength. In the present invention, in order to collect and analyze a large number of light scattering spectra of individual cells, principle component analysis is used as a method for extracting feature quantity of the two-dimensional data. The principal component is extracted by diagonalizing the variance-covariance matrix of the scattered light intensity at each wavelength, and the 1st principal component is an eigenvector belonging to the largest eigenvalue and is a component with the maximum scattering intensity variance. The features of scattered light from the cells are analyzed using the 2nd principal component that is an eigenvector with the second largest eigenvalue, in addition to the 1st principal component, to create a prediction model for classifying the difference in the feature quantities of normal cells and abnormal cells. As a prediction model, specifically, a k-nearest neighbor algorithm (kNN) method, a decision tree method, or a random forest (RF) method is used. As accuracy evaluation, whether cells that are not used in learning are normal cells or abnormal cells is predicted from the feature quantity of light scattering spectrum of the cells using the learned prediction model, and the validity and accuracy of the analysis method are evaluated based on the accuracy rate.

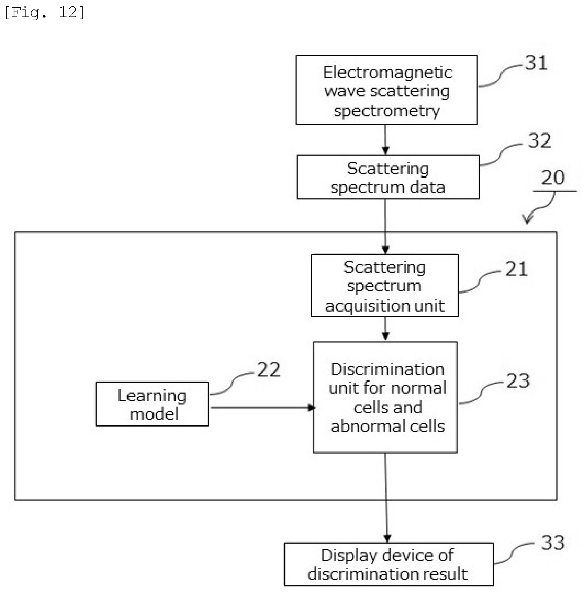

The present invention provides a method for AI analysis by measuring a light scattering spectrum of individual cells contained in a sample collected from a patient, analyzing a feature quantity of the obtained light scattering spectrum by the above method, and discriminating whether the cells contained in the sample are normal cells or abnormal cells using a learning model constructed from previously obtained light scattering spectra. Furthermore, in such AI analysis, as shown in Fig. 12, it is preferable to use a cell discrimination device 20 including a scattering spectrum acquisition unit 21 for obtaining a scattering spectrum 32 by irradiating a sample containing cells collected from a patient with electromagnetic waves by an electromagnetic wave scattering spectrometry device 31, a previously obtained learning model 22 of scattering spectra of normal cells and abnormal cells, and a discrimination unit 23 for discriminating between normal cells and abnormal cells using the learning model 22. The discrimination result by the discrimination unit 23 of the cell discrimination device 20 is, for example, output to a display device 33, such as a display that can classify numerically and display normal cells and abnormal cells (tumor cells). The cell discrimination device 20 includes a computer, the programs of the learning model 22, the scattering spectrum acquisition unit 21, and the discrimination unit 23 are mounted on a memory, and a processor executes the programs.

Hereinafter, examples of an embodiment of the present invention will be described in detail with reference to the drawings. The scope of the present invention is not limited to the following examples and illustrated examples, and many modifications and variations are possible.

[Example 1]

In this Example, it is shown by experiments using cultured animal cells that a change in intracellular structure of submicrometer, which is difficult to be observed with an optical microscope, has a causal relationship with a light scattering spectrum and the light scattering spectrum includes information on the intranuclear microstructure.

A light scattering spectrum of single cells was measured with a dark-field inverted microscope equipped with a spectrometer in a wavelength range of 420 to 720 nm. In this Example, a polymerization inhibitor, Cytochalasin D, is added to cultured animal cells to fragmentate filamentous actin filaments constituting the cytoskeleton, and experiments to examine what changes occurred in the light scattering spectrum of the single cells during this process were conducted. Since the light scattering spectrum of cells includes the shape of white light spectrum of a light source, a light scattering efficiency spectrum (SES) obtained by dividing the actual measurement value in the scattered light measurement by the scattered light intensity from the light source was used as a standard of light scattering spectrum. SES shows wavelength dependency of the scattered light intensity when the intensities of entered light were the same at all wavelengths. The absolute value of SES of each cell greatly varies by the conditions of cells, irradiation conditions of light to cells, adjustment of the object lens for collecting scattered light, and so on. In order to correct this variance in the measurement, normalized light scattering efficiency spectrum ($SES_{n710}$) obtained by normalizing the entire spectrum by the numerical value of SES at 710 nm was used in the analysis by machine learning.

The cells used were a canine renal tubules epithelial cell-derived cell line (MDCK; RCB0995, RIKEN BioResource Research Center). The cells were cultured using Dulbecco's modified Eagle's medium (phenol red-free) supplemented with fetal bovine serum (FBS) and antibiotics on a cell culture dish. The culture medium was replaced by a fresh one just before the cells reach confluence (coverage: 80%), and the cell culture dish was disposed on a sample stage. The center of the cell culture dish was observed by a dark-field inverted microscope (Olympus IX81 with dark-field condenser lens). The cell culture dish 10 was provided with a ring-shaped spacer 11 (thickness: 30 $\mu$m) at the center, a culture medium 12 was added to the area with a diameter of 1.70 cm inside the ring, and MDCK cells 14 were seeded and cultured. In order to prevent drying of the culture medium at the time of observation, a cover glass 13 was placed on the upper surface.

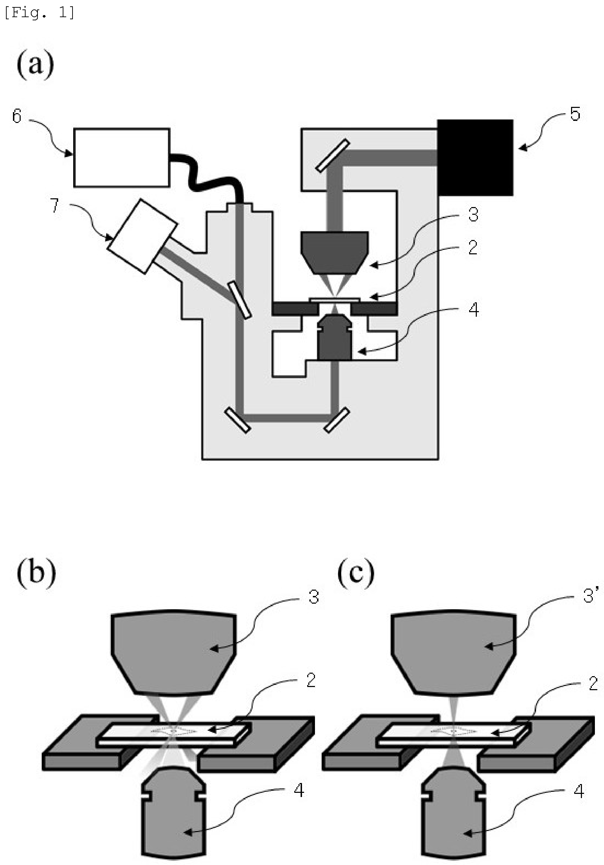

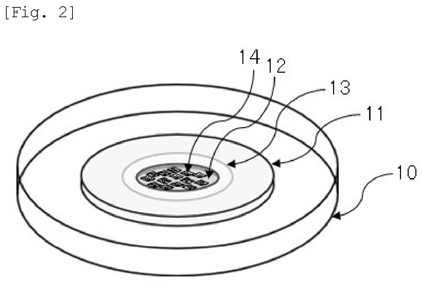

Fig. 1 shows an outline of a measurement system. Fig. 2 shows a configuration of a cell culture dish used in the measurement. As shown in Fig. 1(b), a halogen lamp was used as a lighting source, the cells on the cell culture dish were irradiated with illumination light through a dark-field condenser lens, and the scattered light from the cells was collected by an object lens. Since cells are lighted using a dark-field condenser lens with a numerical aperture larger than the object lens, light collected by the object lens does not include the component of light passed through the cells (transmitted light). In contrast, in an ordinary optical microscope image (bright-field image), as shown in Fig. 1(c), light passed through cells is collected by an object lens.

The scattered light collected by the object lens is divided into two paths, and one is imaged on the CMOS (Complementary Metal-Oxide-Semiconductor) camera to obtain a dark-field image. The other is introduced into a spectroscope through an optical fiber, and the scattered light intensity spectrum measured by the spectroscope is divided by the illumination light spectrum to obtain SES. In the scattered image formed on the optical fiber side, since the core diameter of the optical fiber corresponds to the area with a diameter of about 30 $\mu$m on the cell culture dish, SES of approximately one cell is selectively measured.

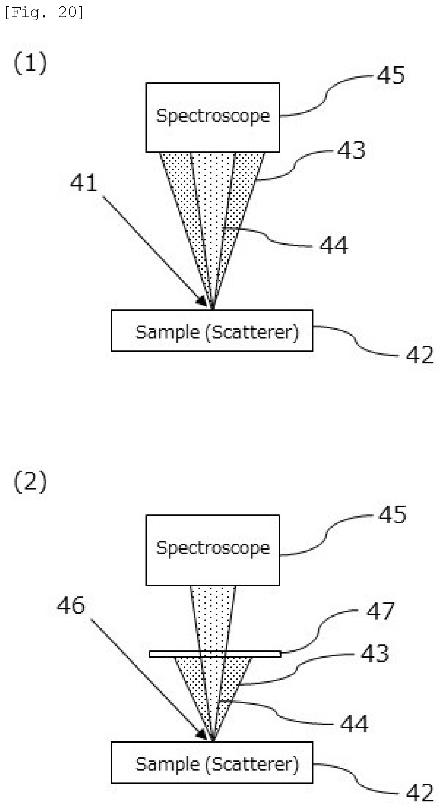

Here, in the spectroscope, in addition to Rayleigh scattering and Mie scattering, Raman scattering is also detected. However, the intensity of Raman scattering is overwhelmingly lower than those of Mie scattering light and Rayleigh scattering light and is lower than the detection limit of the spectroscope, and the intensity is negligible or undetectable in this measurement. That is, as shown in Fig. 20(1), in the present invention, exciting light 41, for example, light containing all wavelengths in the visible range, such as white light, is irradiated to a sample (scatterer). Since Mie scattering light and Rayleigh scattering light 43 as scattered light have significantly high intensities compared to Raman scattering light and fluorescence 44, in the spectroscope 45, the Raman scattering light and fluorescence 44 are ignored as scattering spectrum components. In contrast, as shown in Fig. 20(2), in an optical system for selectively detecting Raman scattering light and fluorescence 44, the main elements that are detected by light scattering are Mie scattering and Rayleigh scattering, if light having strong intensity at a specific wavelength as the exciting light 46 and a strong optical filter (exciting light cut filter) 47 for cutting Mie scattering light and Rayleigh scattering light are not used. The Mie scattering light and the Rayleigh scattering light have the same wavelengths as the irradiation light (exciting light) to be irradiated to cells, but the Raman scattering light and fluorescence have wavelengths different from that of the exciting light.

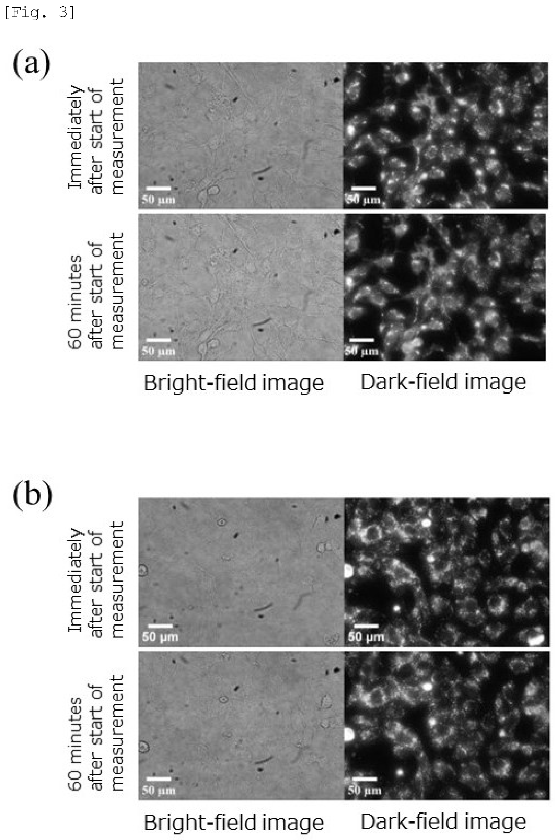

Fig. 3 shows observation results of the bright-field image and the dark-field image of MDCK cells observed by a CMOS camera. Fig. 3(a) is images of MDCK cells when Cytochalasin D was not added, and Fig. 3(b) is images of MDCK cells when Cytochalasin D was added and cytoskeleton was fragmented. Regarding the bright-field images and the dark-field images of Fig. 3(a) and (b), a significant difference cannot be visually observed between images immediately (0 minute) and 60 minutes after addition of Cytochalasin D and mounting under a microscope. The appearance of actual fragmentation of actin filaments by adding Cytochalasin D under the above conditions was verified by separately observing a sample stained with a fluorescent dye (SPY555-actin, SPIROCHROME, SC202) with a confocal microscope. The actin filaments constituting cytoskeleton do not have absorption in the visible light region and have a diameter of 10 nm or less. Accordingly, observation with an optical microscope is difficult, and the results that fragmentation of actin filaments cannot be observed in the bright-field images of the photographs on the left side in Fig. 3(a) and (b) are reasonable. In the dark-field images of the photographs on the right side in Fig. 3(a) and (b), fragmentation of actin filaments may be observed, but a clear difference cannot be visually found. However, it is possible to classify cells in which actin filaments are fragmented by quantifying the structural change by applying analysis using machine learning described later.

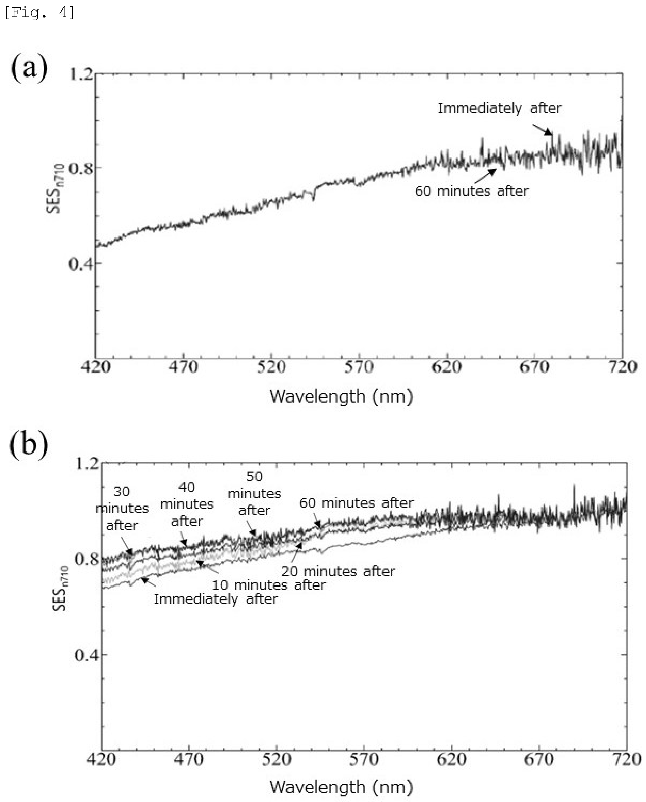

Fig. 4(a) shows comparison of SES of MDCK cells in a culture medium not containing Cytochalasin D 0 minutes and 60 minutes after the arrangement on the microscope. Both $SES_{n710}$ immediately after (0 minutes after) and 60 minutes after the start of measurement were almost identical. Even when Cytochalasin D was not added, there was concern that the structure of the cells might change by being left for a long time to change the $SES_{n710}$, but this experiment demonstrated that in this experimental condition, no structural change of cells is observed in the light scattering spectrum within at least 60 minutes.

Then, Cytochalasin D adjusted to a concentration of 200 $\mu$M was added to the MDCK cells to inhibit polymerization of actin filaments, and SES of the cells was measured every 10 minutes from immediately after the addition until 60 minutes later. The change in $SES_{n710}$ is shown in Fig. 4(b). From the results of Fig. 4(b), a change in the light scattering spectrum was recognized from immediately after the addition of Cytochalasin D. According to this result, it was recognized that the light scattering intensity tended to increase with time on the short light scattering wavelength side from around 500 nm. It is believed that the light scattering by fragments of actin filaments with various lengths due to inhibition of polymerization of the actin filaments contributes to Rayleigh scattering. The increase in the scattering intensity on the short wavelength side observed in Fig. 4(b) can be explained by this contribution. Thus, in this Example, the light scattering spectrum of visible light has a causal relationship with a structural change in the cell, and it is clearly demonstrated that the difference in the intracellular structure, which cannot be clearly discriminated by the bright-field image and the dark-field image, can be measured.

As shown in Fig. 4, the light scattering spectrum of unstained transparent cells with visible light exhibits features of a flat spectrum not having particularly clear features. However, the spectrum includes not only the light scattering by the actin filaments of the cytoskeleton but also light scattering by the microstructure, cell nuclei, and intranuclear microstructure of cells. It is believed that as a result of comprehensive reflection of all of these light scatterings, the spectrum appears flat overall, but there is a possibility that a slight irregularity that looks like noise on the flat spectrum includes information having a causal relationship with these microstructures. However, it is almost impossible to predict this irregularity physically and definitely. Accordingly, in the present invention, as a solution to this, information originated from the microstructure in a cell is analyzed from the light scattering spectrum by AI analysis based on machine learning not requiring physical and definite prediction.

[Example 2]

This Example shows results clearly demonstrating that a light scattering spectrum of which interpretation is significantly difficult from a physics perspective is discriminated by machine learning using pathological specimens prepared by fixing and staining normal cells and tumor cells obtained from actual patients according to a flow of known pathological diagnosis.

Pleural effusion was collected with a syringe from the upper edge of ribs of each of normal specimens (A to F) and specimens (G, H, and I) containing cells suspected to be suffering from pleural mesothelioma as subjects, and the cells contained in each pleural effusion were smeared on a slide glass and then spray-fixed to be fixed in 95% alcohol fixative. The staining was performed according to Papanicolaou staining procedure: cell nuclei were stained with Hematoxylin, and cytoplasm stained with OG-6 staining solution and then with EA-50 staining solution. The stained cells were permeated in a xylol tank, and the finally sealed sample was used as the specimen. The cells in each specimen were discriminated by a pathologist, and a light scattering spectrum of a specimen prepared by making individual cells discriminated to pleural mesothelium cells as normal cells and other tumor cells was measured.

Regarding normal cells, 23 to 25 previously marked normal cells contained in the specimens A to F were subjected to measurement, and regarding tumor cells, 13 to 27 previously marked tumor cells contained in the specimens G to I were subjected to measurement. Here, the tumor cells include mesothelioma cells and also adenocarcinoma cells. Furthermore, the possibility that the cells classified into normal cells or tumor cells including also reactive mesothelial cells cannot be eliminated.

One characteristic of mesothelioma cells is that the probability that the number of nuclei in a cell is two is higher than other adenocarcinoma cells and reactive mesothelial cells. In addition, characteristically, mesothelioma cells and adenocarcinoma cells easily form cell aggregates and can be discriminated by isolating reactive mesothelial cells.

However, when the sizes of nuclei are simply compared, the size of nuclei of mesothelioma cells is located between the adenocarcinoma cells and reactive mesothelial cells, but since there is a distribution in the sizes of nuclei, it is difficult to discriminate them without observing the distribution of nuclear sizes in a large number of cells, rather than by discrimination by observing individual cell nuclei. The nuclei of mesothelioma cells are present in the center of the cells and the nuclei of adenocarcinoma cells are often ubiquitous at the edges of the cells but cannot be discriminated from reactive mesothelial cells by only the position of nuclei.

This Example has a purpose of verifying that the light scattering spectrum of tumor cells is different from the light scattering spectrum of normal cells, and does not have a purpose of discriminating adenocarcinoma cells and reactive mesothelial cells from mesothelioma cells. That is, whether cells in a specimen are mesothelioma cells or normal is determined by discrimination from both pathological and clinical perspectives, and the present invention is intended solely for the purpose of detecting abnormal cells different from normal cells.

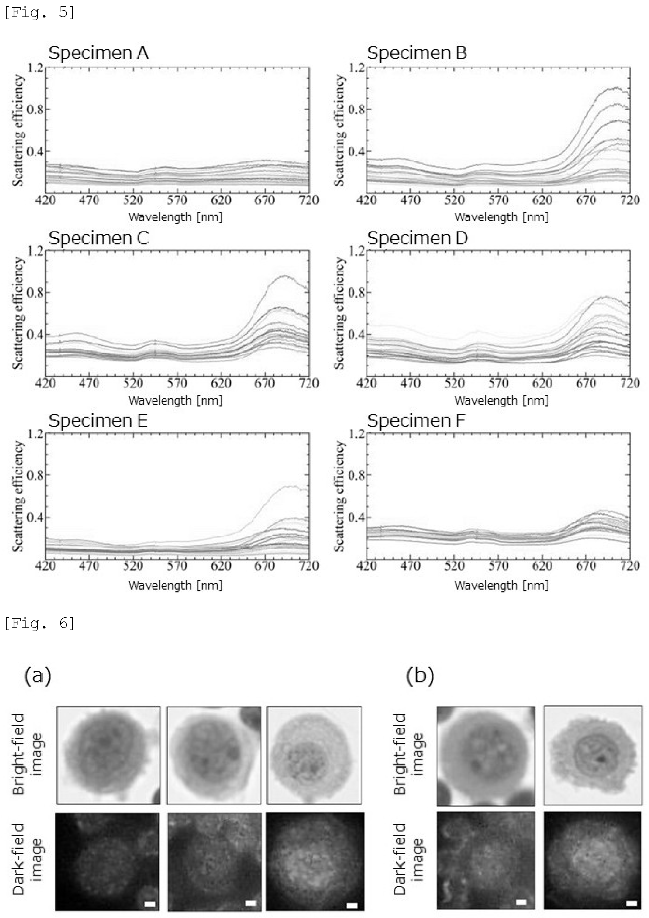

Fig. 5 shows light scattering efficiency spectra (SES) measured for cells contained in normal specimens A to F. The features of SES of cells contained in the respective specimens are different from each other. Although the SES of cells in specimens A and F are mostly flat spectra, cells contained in specimens B, C, and D characteristically include cells having a peak of scattering efficiency at around 700 nm. In specimen E, a peak at about 700 nm was observed only in SES of a part of measured cells, but SES of the remaining cells were almost flat spectra.

SES of cells contained in specimens A to F were measured, and Fig. 6 shows (a) bright-field images (top) and dark-field images (bottom) of cells in which a scattering peak was observed at around 700 nm and (b) bright-field images (top) and dark-field images (bottom) of cells of which SES was flat. In the cells of specimen B, it was confirmed that the nucleus size of the cells that exhibited a scattering peak was large, but in the cells of specimen C, a clear relationship was not recognized between the size of nuclei and the scattering peak. Consequently, it was demonstrated that there is a possibility that the appearance of a scattering peak is influenced by not only enlargement of the nucleus size but also another factor such as a difference in relative refractive index due to ease of staining or the like.

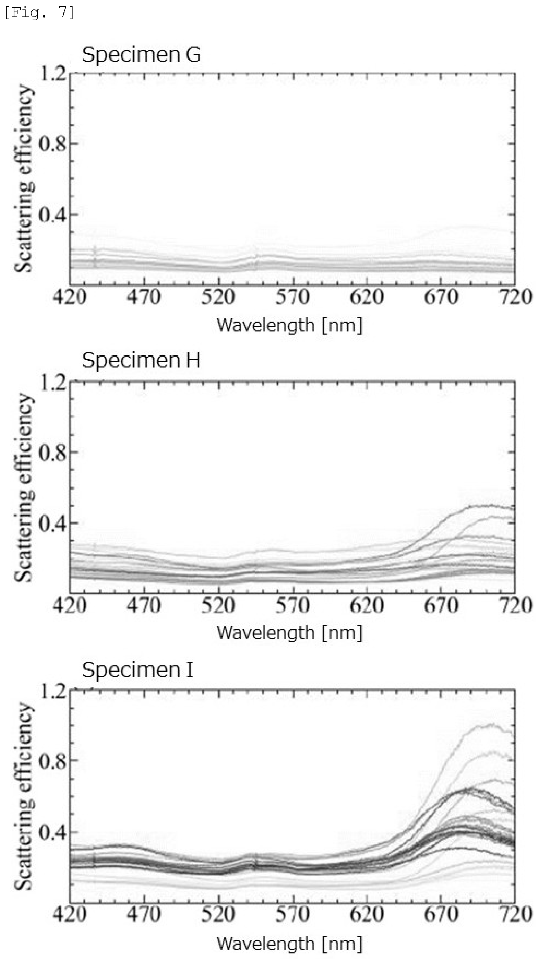

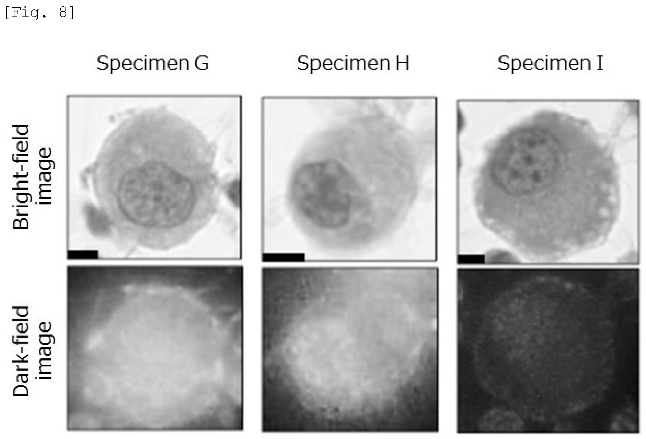

Fig. 7 shows the results of measurement of SES of cells contained in specimens G to I suspected to be suffering from mesothelioma. Fig. 8 shows optical microscope images (bright-field images (top) and dark-field images (bottom)) of cells contained in the respective specimens.

In Fig. 6, all cells contained in specimen G exhibited flat SES, a small number of cells contained in specimen H exhibited SES having a peak at near 700 nm, and a large number of cells contained in specimen I exhibited SES having a peak at near 700 nm. In the optical microscope images of the cells shown in Fig. 8, there is a distribution of the size of the nuclei included in the respective cells, and both central and ubiquitous nucleus locations were observed. The above results demonstrated that SES clearly discriminates cells showing a peak at near 700 nm and cells showing a flat shape, but it was demonstrated that normal cells and tumor cells are not necessarily discriminated only by the above results. It is believed that this SES of the cytologic specimen is reflected by, in addition to the microstructures of cells, cell nuclei, cytoskeleton, and intracellular organelles, causal relationships with reflection, absorption, and refraction of a dye bound to the cells by Papanicolaou staining. It is nearly impossible to analyze all of these considerations included in SES by physics-based theories. In the present invention, as the discrimination method, machine learning, which is numerical analysis that is not based on physical logic, is used.

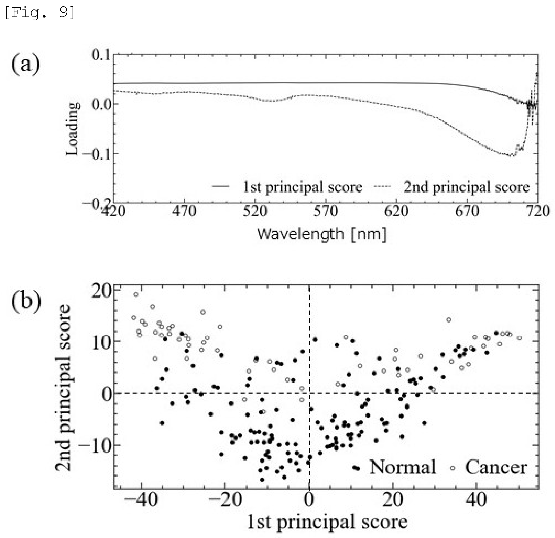

Normalized light scattering efficiency spectra ($SES_{n710}$) of cells contained in specimens A to I were subjected to principal component analysis to obtain loadings of the 1st principal component and the 2nd principal component shown in Fig. 9(a). From these loadings, percentages (scores) of the 1st principal component and the 2nd principal component included in the $SES_{n710}$ of individual cells are determined, and the scatter diagram shown in Fig. 9(b) is obtained by plotting the scores of the 1st principal component on the horizontal axis and the scores of the 2nd principal component on the vertical axis. For example, it is meant that $SES_{n710}$ in which both scores of the 1st principal component and the 2nd principal component are present in the plus region (Fig. 9(b): first quadrant) can be expressed by addition of the loadings of the 1st principal component and the 2nd principal component and that $SES_{n710}$ in which the 1st principal component and the 2nd principal component are present in the plus region and the minus region, respectively (Fig. 9(b): second quadrant) can be expressed by subtracting the loading of the 2nd principal component from the loading of the 1st principal component. $SES_{n710}$ close to the origin means that the 1st principal component and the 2nd principal component are almost not included. The filled circles are scores corresponding to $SES_{n710}$ of normal cells contained in normal specimens A to F, and the open circles are scores corresponding to $SES_{n710}$ of tumor cells contained in specimens G to I suspected to be suffering from mesothelioma. The scores of normal cells are distributed mainly in the third quadrant and the fourth quadrant, but also in the first quadrant and the second quadrant, and the scores of tumor cells are concentrated in the first quadrant and the second quadrant. The results demonstrate that normal cells and tumor cells that cannot be clearly classified by SES of Figs. 5 and 7 can be numerically classified by the 2nd principal component score.

Here, the method for determining the score of each principal component will be described. The score of the 1st principal component of a patient n (n = 1 to N) is shown by the following equation 1. As shown in the following equation 2, $w_1(\lambda)$ is determined such that the sum of squares of $w_1(\lambda)$ is 1 by weighting (eigenvector) of the 1st principal component, and as shown in the following equation 3, the $w_1(\lambda)$ at which the variance $V_1$ of the score of the 1st principal component becomes maximum is determined.
[Equation 1]

$$Z_1^n = \sum_\lambda w_1(\lambda)X^n(\lambda) \quad \cdots \text{(Equation 1)}$$

[Equation 2]

$$|w_1| = \sum_\lambda \sqrt{\{w_1(\lambda)\}^2} = 1 \quad \cdots \text{(Equation 2)}$$

[Equation 3]

$$V_1 = \frac{1}{N}\sum_{n=1}^{N}(Z_1^n - \overline{Z_1})^2 \quad \cdots \text{(Equation 3)}$$

The score of the 2nd principal component of a patient n (n = 1 to N) is shown by the following equation 4. As in the 1st principal component, $w_2(\lambda)$ is determined such that the sum of squares of $w_2(\lambda)$ is 1, as shown in the following equation 5, by weighting the 2nd principal component, and as shown in the following equation 6, the $w_2(\lambda)$ is imposed with an orthogonal condition so that the $w_2(\lambda)$ is orthogonal to the $w_1(\lambda)$, and the $w_2(\lambda)$ at which the variance of the score of the 2nd principal component becomes maximum under this condition is determined.
[Equation 4]

$$Z_2^n = \sum_\lambda w_2(\lambda)X^n(\lambda) \quad \cdots \text{(Equation 4)}$$

[Equation 5]

$$|w_2| = \sum_\lambda \sqrt{\{w_2(\lambda)\}^2} = 1 \quad \cdots \text{(Equation 5)}$$

[Equation 6]

$$w_1(\lambda) \cdot w_2(\lambda) = \sum_\lambda w_1(\lambda)\,w_2(\lambda) = 0 \quad \cdots \text{(Equation 6)}$$

In the score of the $n^{th}$ principal component of a patient n (n = 1 to N), the $w_n(\lambda)$ at which the variance of the score of the $n^{th}$ principal component becomes maximum is determined by adding the orthogonal condition. Thus, spectrum data are much simpler than image data, and less arbitrary analysis is possible.
The type of cells that are not known, whether they are normal cells or tumor cells, can be estimated by comparing the $SES_{n710}$ of the cells to a scatter diagram created as in Fig. 9(b). The scores of loadings of the 1st principal component and the 2nd principal component shown in Fig. 9(a) are determined in the $SES_{n710}$ obtained for unknown cells, and whether the unknown cells are normal cells or tumor cells was estimated based on where the scores were plotted on the graph of Fig. 9(b). For example, when the score of unknown cells is in the third quadrant or fourth quadrant where a large number of scores of normal cells are present, the unknown cells are likely to be normal cells, and when the score is in the first quadrant or second quadrant where a large number of scores of tumor cells are present, the unknown cells are likely to be tumor cells.

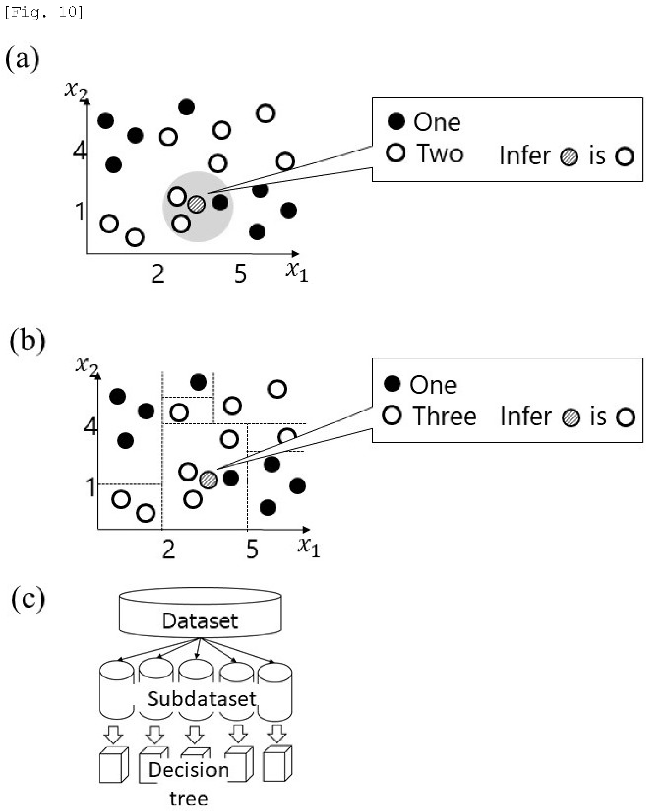

Examples of this binary classification method for determining whether cells are normal cells or tumor cells include a k-nearest neighbor algorithm method, a decision tree method, and a random forest method. As shown in Fig. 10(a), the k-nearest neighbor algorithm method is a method of classifying a sample into which class it belongs by taking a majority vote among the nearest k samples. As shown in Fig. 10(b), the decision tree method is a method of dividing a scatter diagram into sections (decision tree) and classifying a sample by majority vote within each section. As shown in Fig. 10(c), the random forest method is a method of classifying a sample by taking a majority vote among multiple different decision trees and further taking a majority vote of the results of those votes.

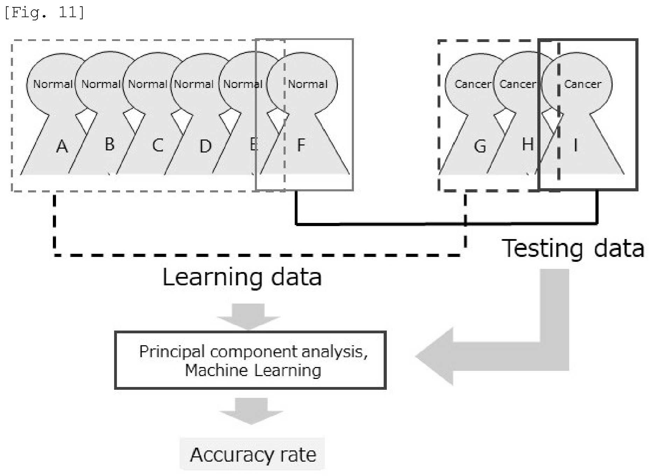

As shown in Fig. 11, the $SES_{n710}$ of normal cells, which were assumed as unknown cells, contained in one specimen among specimens A to F was used as test data, the $SES_{n710}$ of the remaining normal cells was used as prediction model learning data for creating a scatter diagram, the $SES_{n710}$ of tumor cells, which were assumed as unknown cells, contained in one specimen among specimens G to I was used as test data, and the $SES_{n710}$ of the remaining tumor cells was used as learning data for creating a scatter diagram, and the test data of the normal cells and the tumor cells were binarily classified by the above method. As a result, the accuracy rate of the k-nearest neighbor algorithm method, the decision tree method, and the random forest method were 69.5%, 70.9%, and 72.4%, respectively, and it was demonstrated that these analysis methods can numerically classify normal cells and tumor cells based on $SES_{n710}$. Thus, this Example clearly demonstrates that light scattering spectra, which are significantly difficult to interpret from a physics perspective, of normal cells and tumor cells obtained from actual patients can be discriminated by machine learning.

In this analysis, only the 1st principal component and the 2nd principal component were considered, but the classification accuracy rate between normal cells and tumor cells can be increased, for example, from the microscopic shape characteristics of a light scattering spectrum that cannot be visually detected by humans by proceeding with classification while considering further higher-order principal components to increase the accuracy. Furthermore, further higher accuracy can be expected by analyzing the vector information on a dark-field image shown in Fig. 3, the frequency information obtained by two-dimensional Fourier expansion, and so on by the same method as in the light scattering spectrum and incorporating the information as a feature quantity in combination with the light scattering spectrum into the learning model.

In the pathological diagnosis, rather than discriminating whether individual cells contained in a pathological specimen are normal or abnormal, an important purpose is to diagnose whether abnormal cells are contained in a pathological specimen or not. For example, even if the discrimination accuracy (accuracy rate) of individual cells is 70%, if it is possible to diagnose with nearly 100% accuracy that abnormal cells are contained in a pathological specimen by discriminating several hundreds of cells contained in a cytologic specimen and statistically analyzing the result thereof, the purpose is achieved with a high level. In order to improve the diagnostic accuracy of a pathological specimen, not only the discrimination accuracy of one cell but also the measurement speed of a light scattering spectrum, measurement accuracy, and so on are important. Furthermore, it is believed that the diagnosis accuracy can be overwhelmingly improved when an overwhelmingly large number of normal cells can be removed before measurement or when there is estimated information on how many abnormal cells are in a specimen. Thus, this Example clearly demonstrates a possibility of realizing a diagnosis of a pathological specimen with high sensitivity and high accuracy that surpasses conventional methods.

[Example 3]

This Example shows the results of discrimination by, as in Example 2, using a pathological specimen obtained by fixing and staining normal cells and tumor cells obtained from actual patients according to a flow of a known pathological diagnosis and, different from Example 2, performing machine learning of the light scattering spectrum also considering higher order principal components.

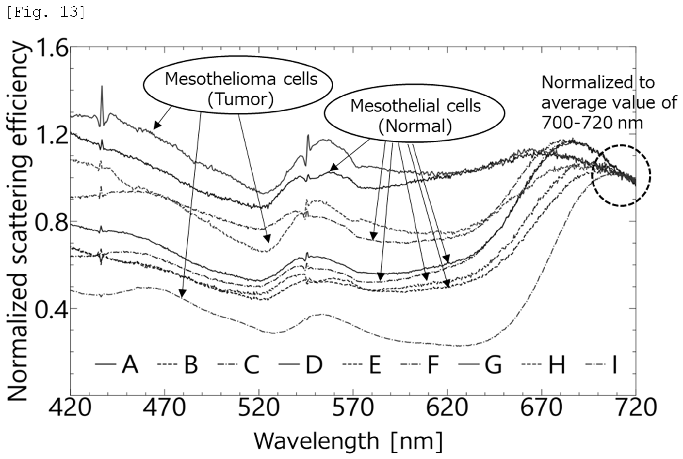

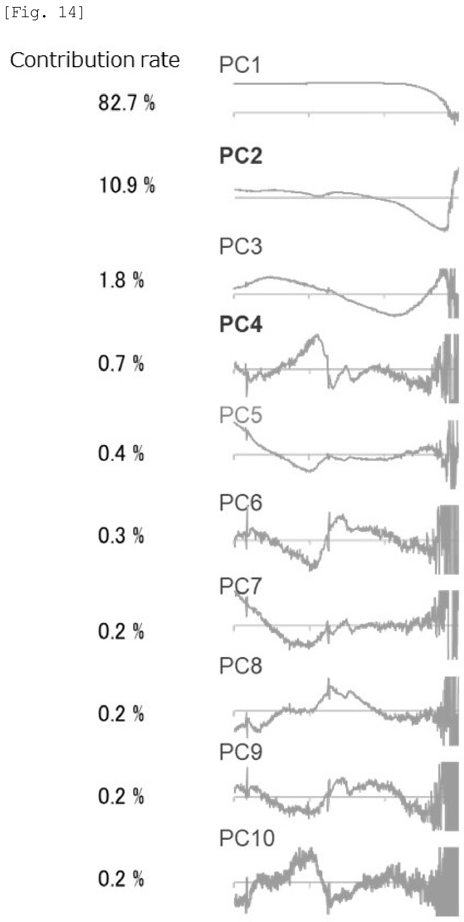

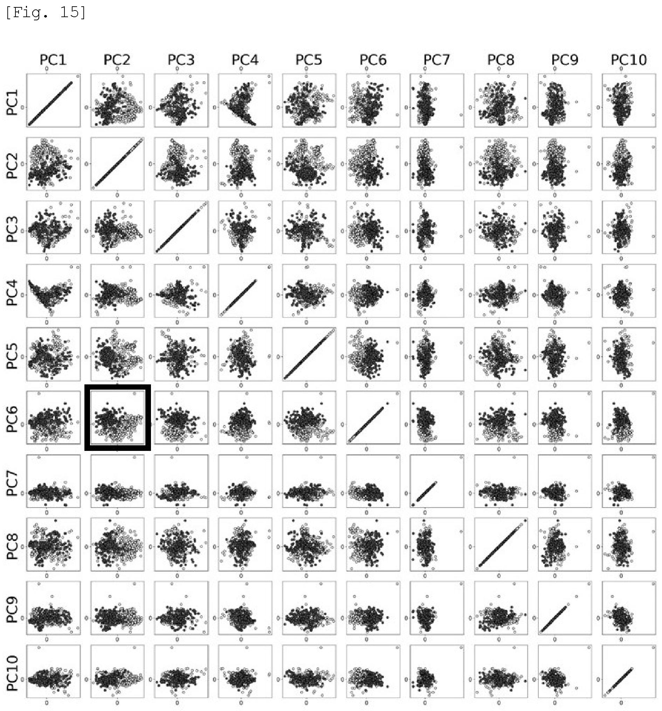

As in Example 2, loadings of the 1st principal component to the 10th principal component are obtained by principal component analysis of normalized light scattering efficiency spectra ($SES_{n710}$) of cells contained in specimens A to I (see Fig. 13). Fig. 14 shows the contribution rates and loading results of the 1st principal component (PC1), the 2nd principal component (PC2), ..., and the 10th principal component. From these loadings, the percentages (scores) of the $n$th principal component ($n$ = 1 to 10) and the $m$th principal component ($m$ = 1 to 10) contained in $SES_{n710}$ of individual cells were determined. Fig. 15 shows groups of plots (scatter diagrams) with the scores of the $n$th principal component on the horizontal axis and the scores of the $m$th principal component on the vertical axis. Fig. 15 shows 100 scatter diagrams including plots of the same principal components. Among them, since swapping the vertical axis and horizontal axis of each principal component will yield essentially the same information, 45 scatter diagrams (upper right of the matrix of Fig.

15) wherein n < m were verified with the n$^{th}$ principal component and the m$^{th}$ principal component, and it was demonstrated that the difference between normal cells and tumor cells can be clearly discriminated by using the scatter diagram of the scores of the 2nd principal component (PC2) and the 6$^{th}$ principal component (PC6).

A clear difference between normal cells and tumor cells appears in the scatter diagram of the scores of the 2nd principal component and the 6$^{th}$ principal component, but there is room for clear differences in other components by changing the type of the cells or adjusting the analysis parameter.

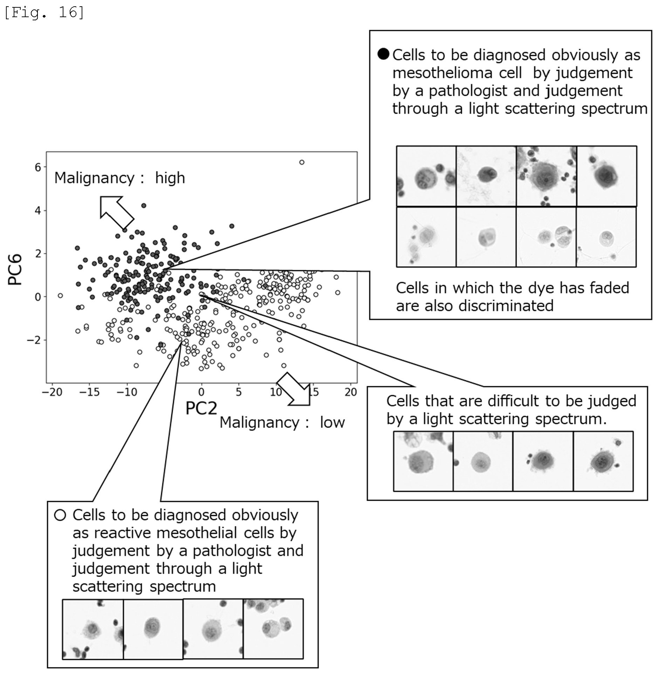

As described above, it was demonstrated that the difference between normal cells and tumor cells can be clearly discriminated by using the scatter diagram of scores of the 2nd principal component and the 6$^{th}$ principal component. At the same time, a possibility that the scatter diagram of scores of the 2nd principal component and the 6$^{th}$ principal component reflects the malignancy of tumor cells is suggested. That is, as shown in Fig. 16, the difference between tumor cells and normal cells is not a simple yes/no, there is also an intermediate state between yes and no, and there is a possibility of showing the intermediate state as the malignancy of the tumor cells. There are cells that are diagnosed clearly to be mesothelioma by the judgement by a pathologist and also by the judgement through a light scattering spectrum (of these cells, cells in which the dye has faded are also discriminated), cells of which the scores are near the origin and difficult to be judged by a light scattering spectrum, and cells to be diagnosed obviously as reactive mesothelial cells by judgement by a pathologist and judgement through a light scattering spectrum, and the malignancy of tumor cells can be shown such that the more to the upper left, the higher the malignancy, and the more to the lower right, the lower the malignancy.

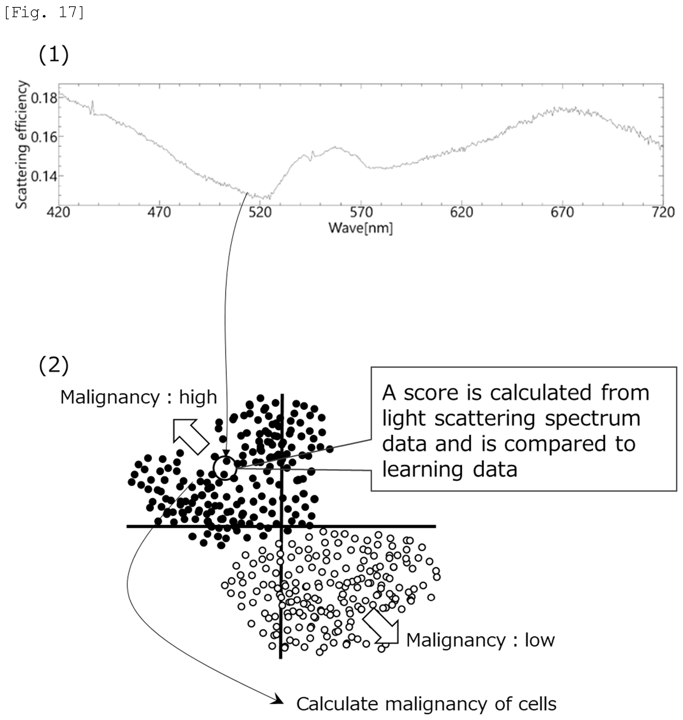

Thus, by establishing learning data, when a pathologist is unsure of a decision, pathological diagnosis by image data can be supported by referring to the result of discrimination by light scattering spectra. In addition, if it is possible to accumulate learning data, the accuracy by discrimination by light scattering spectra can be further improved, and full automation not relying on a pathologist can be finally expected. For example, as shown in Fig. 17(1), light scattering spectrum data are obtained from unknown cells (pathologists diagnose by image data of unknown cells based on the information on the shape of the cells of 1 $\mu$m or more), and information on intracellular structure of 1 $\mu$m or less can be obtained from the light scattering spectrum. As shown in Fig. 17(2), a score is calculated from light scattering spectrum data and is compared to learning data to calculate the malignancy of the cells, which supports pathological diagnosis. Score calculation of the light scattering spectrum data is simple compared to diagnosis by image data, and tumor cells can be discriminated with high sensitivity and high accuracy. The information included in image data is different from the information included in light scattering spectrum data, and therefore an improvement in complementary diagnosis accuracy can be expected. An improvement in the judgement accuracy of the malignancy of cells can be expected by combining the learning data of images and learning data of light scattering spectra.

[Example 4]

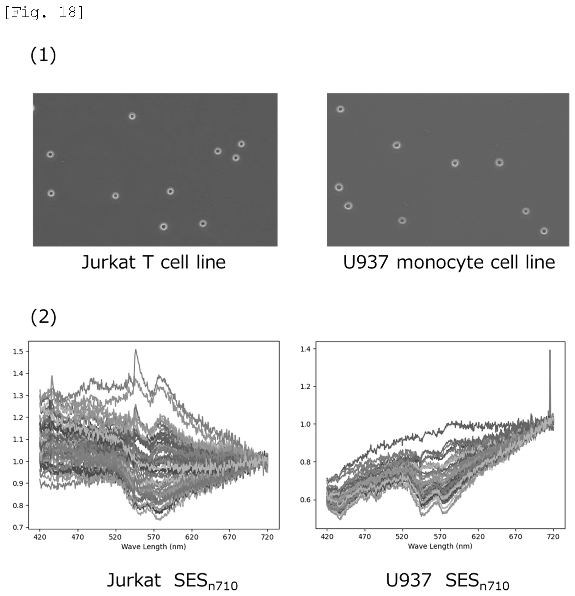

This Example shows a result by discriminating living cells that are not stained as a pathological specimen by the light scattering spectrum. Here, the optical microscope images of two types of living cells of Jurkat T cell line and U937 monocyte cell line and the normalized light scattering efficiency spectra (SES$_{n710}$) of individual cells were obtained. In the optical microscope images shown in Fig. 18(1), it is significantly difficult to discriminate these two types of cells by the characteristics of the shape. In the normalized light scattering efficiency spectra shown in Fig. 18(2), there appears to be a difference in shape between the upper spectra, but the difference is less clear in the lower spectra.

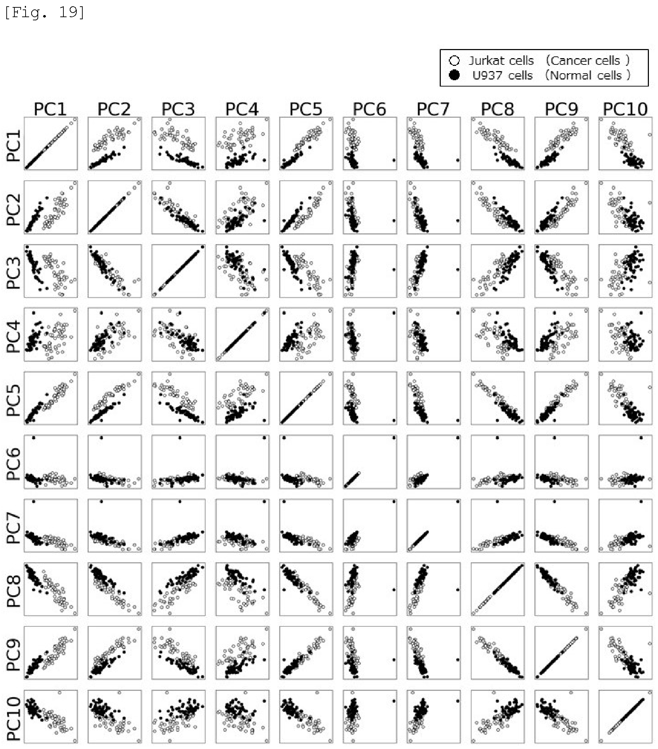

The normalized light scattering efficiency spectra (SES$_{n710}$) shown in Fig. 18(2) were subjected to principal component analysis to obtain the loadings of the 1st principal component to the 10$^{th}$ principal component, and the scores of the n$^{th}$ principal component (n = 1 to 10) and the m$^{th}$ principal component (m = 1 to 10) contained in SES$_{n710}$ of individual cells were determined to obtain the scatter diagrams of Fig. 19. In almost all principal components, different scores were obtained in two types of cells, all of the principal components up to the 10$^{th}$ (PC10) were able to clearly separate two types of cells. The results of this Example were better than those of Examples 2 and 3 that used stained specimens, and it was clearly demonstrated that the present invention can be applied also to living cells. Clear discrimination was possible in unstained living cells, which proves hypothetical validity shown in Example 1.

[Industrial Applicability]

According to the present invention, discrimination of tumor cells, which are conventionally difficult to be discriminated by diagnosis through optical microscope observation by a pathologist, can be easy, and there is a possibility of diagnosis not depending on the experience and degree of proficiency of a pathologist and realization of high accuracy of the diagnosis. Furthermore, comprehensive information on the characteristics of cells can be obtained with higher sensitivity than an optical microscope image and at a higher speed by systematizing the measurement of light scattering spectra, and there is a possibility of realizing full automation and higher speed of pathological diagnosis.

[Reference Signs List]

| | |
|---|---|
| 1 | Microscopic scattering measurement system |
| 2 | Cell culture dish or specimen |
| 3 | Dark-field condenser lens |
| 3' | Bright-field condenser lens |
| 4 | Object lens |
| 5 | Light source (halogen lamp) |
| 6 | Spectroscope (spectrometer) |
| 7 | CMOS camera |
| 10 | Cell culture dish |
| 11 | Spacer |
| 12 | Culture medium |
| 13 | Cover glass |
| 14 | Cultured cells |
| 20 | Cell discrimination device |
| 21 | Scattering spectrum acquisition unit |
| 22 | Learning model |
| 23 | Discrimination unit for normal cells and abnormal cells |
| 31 | Electromagnetic wave scattering spectrometry device |
| 32 | Scattering spectrum |
| 33 | Display device of discrimination result |
| 41,46 | Exciting light |
| 42 | Sample (scatterer) |
| 43 | Mie scattering light and Rayleigh scattering light |
| 44 | Raman scattering light (or fluorescence) |
| 45 | Spectroscope |
| 47 | Optical filter (exciting light cut filter) |

**Claims**

1. A cell discrimination method for discriminating between normal cells and abnormal cells by irradiating a sample containing cells collected from a patient with electromagnetic waves, obtaining an electromagnetic wave scattering spectrum of the sample, and using a learning model obtained by learning features of electromagnetic wave scattering spectra of normal cells and abnormal cells.

2. A cell discrimination method for discriminating between normal cells and abnormal cells by irradiating a sample containing cells collected from a patient with electromagnetic waves including all wavelengths in a specific wavelength region, obtaining electromagnetic wave scattering spectra through Rayleigh scattering and Mie scattering from the sample, and using a learning model obtained by learning electromagnetic wave scattering spectra of normal cells and abnormal cells.

3. The cell discrimination method according to claim 1 or 2, wherein

   the electromagnetic wave scattering spectrum is two-dimensional data showing a relationship of scattered electromagnetic wave intensity with respect to the wavelength of the electromagnetic waves, and
   the learning model learns the two-dimensional data and classifies difference between normal cells and abnormal cells.

4. The cell discrimination method according to claim 3, wherein the learning model extracts a feature quantity of the two-dimensional data using principal component analysis, extracts a principal component by diagonalizing a variance-covariance matrix of scattering intensities at each wavelength, and classifies a difference between feature quantities of normal cells and abnormal cells.

5. The cell discrimination method according to claim 4, wherein in the principal component analysis,

a 1st principal component is an eigenvector with a largest eigenvalue and is a component with a largest variance of scattering intensity,

a 2nd principal component is an eigenvector with a second largest eigenvalue and is a component being orthogonal to the 1st principal component and having a largest variance of scattering intensity, and

an $n^{th}$ principal component is an eigenvector with an $n^{th}$ largest eigenvalue and is a component being orthogonal to the $(n-1)^{th}$ principal component and having a largest variance of scattering intensity.

6. The cell discrimination method according to claim 1 or 2, wherein the sample is a sample fixed and stained with alcohol or formalin.

7. The cell discrimination method according to claim 1 or 2, wherein the sample is a living cell.

8. The cell discrimination method according to claim 1 or 2, wherein the electromagnetic wave scattering spectrum is a forward scattering spectrum.

9. The cell discrimination method according to claim 1 or 2, wherein the electromagnetic waves to be irradiated have a wavelength of 100 nm to 1 $\mu$m.

10. The cell discrimination method according to claim 1 or 2, wherein the electromagnetic waves to be irradiated are visible light.

11. The cell discrimination method according to claim 1, wherein the electromagnetic wave scattering spectrum is one or both of scattering spectra by Rayleigh scattering and Mie scattering.

12. The cell discrimination method according to claim 2 or 11, wherein the difference between the electromagnetic wave scattering spectra of normal cells and abnormal cells is due to an increase or decrease of scattering caused by a change in size of cell structure when the change is smaller than or equal to a wavelength of visible light.

13. The cell discrimination method according to any of claims 1, 2, and 11, wherein the abnormal cells are tumor cells.

14. The cell discrimination method according to claim 13, wherein the tumor cells are cancer cells.

15. The cell discrimination method according to claim 1 or 2, wherein the electromagnetic waves to be irradiated are ultraviolet rays or X-rays.

16. The cell discrimination method according to claim 1 or 2, wherein the electromagnetic waves to be irradiated are infrared light rays.

17. A cell discrimination device comprising a scattering spectrum acquisition unit for obtaining an electromagnetic wave scattering spectrum by irradiating a sample containing cells collected from a patient with electromagnetic waves, a learning model obtained by learning electromagnetic wave scattering spectra of normal cells and abnormal cells, and a discrimination unit for discriminating between normal cells and abnormal cells by using the learning model.

18. A cell discrimination device comprising an irradiation unit for irradiating a sample containing cells collected from a patient with electromagnetic waves having all wavelengths in a specific wavelength region, an electromagnetic wave scattering spectrum acquisition unit for obtaining Rayleigh scattering and Mie scattering, a learning model obtained by learning electromagnetic wave scattering spectra of normal cells and abnormal cells, and a discrimination unit for discriminating between normal cells and abnormal cells by using the learning model.

[Fig. 1]

(a)

(b)

(c)

[Fig. 2]

[Fig. 3]

(a)

Bright-field image    Dark-field image

(b)

Bright-field image    Dark-field image

[Fig. 4]

(a)

(b)

[Fig. 5]

[Fig. 6]

[Fig. 7]

Specimen G

Specimen H

Specimen I

[Fig. 8]

|  | Specimen G | Specimen H | Specimen I |

Bright-field image / Dark-field image

[Fig. 9]

[Fig. 10]

(a)

(b)

(c)

[Fig. 11]

[Fig. 12]

[Fig. 13]

[Fig. 14]

[Fig. 15]

[Fig. 16]

● Cells to be diagnosed obviously as mesothelioma cell by judgement by a pathologist and judgement through a light scattering spectrum

Cells in which the dye has faded are also discriminated

Malignancy : high

Malignancy : low

Cells that are difficult to be judged by a light scattering spectrum.

○ Cells to be diagnosed obviously as reactive mesothelial cells by judgement by a pathologist and judgement through a light scattering spectrum

[Fig. 17]

(1)

(2)

Malignancy : high

A score is calculated from light scattering spectrum data and is compared to learning data

Malignancy : low

Calculate malignancy of cells

[Fig. 18]

(1)

Jurkat T cell line

U937 monocyte cell line

(2)

Jurkat $SES_{n710}$

U937 $SES_{n710}$

[Fig. 19]

[Fig. 20]

(1)

(2)

## INTERNATIONAL SEARCH REPORT

| International application No. |
|---|
| **PCT/JP2024/027247** |

| A. | CLASSIFICATION OF SUBJECT MATTER |
|---|---|

*G01N 21/27*(2006.01)i; *G01N 33/48*(2006.01)i; *G01N 33/483*(2006.01)i
FI: G01N21/27 E; G01N33/483 C; G01N33/48 M

According to International Patent Classification (IPC) or to both national classification and IPC

| B. | FIELDS SEARCHED |
|---|---|

Minimum documentation searched (classification system followed by classification symbols)

G01N21/00-G01N21/61; G01N33/48-G01N33/98; G01N15/00-G01N15/1492; C12M1/00-C12M3/10; C12N1/00-C12N7/08; C12Q1/00-C12Q3/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | EP 2887050 A1 (HOCHSCHULE REUTLINGEN) 24 June 2015 (2015-06-24) paragraphs [0021]-[0024], [0031]-[0035], [0039], [0042]-[0052], fig. 1-4 | 1-3, 6-18 |
| Y | | 4-5 |
| X | US 2010/0145199 A1 (JOHNSON KRISTIE) 10 June 2010 (2010-06-10) paragraphs [0032]-[0069], fig. 1-8 | 1, 3, 6-7, 9-10, 13-14, 17 |
| Y | | 4-5 |
| Y | JP 2005-534899 A (PFIZER INC.) 17 November 2005 (2005-11-17) paragraphs [0024]-[0027] | 4-5 |
| A | CN 111024624 A (SOUTHEAST UNIVERSITY) 17 April 2020 (2020-04-17) entire text, all drawings | 1-18 |

☐ Further documents are listed in the continuation of Box C.      ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **27 September 2024** | **08 October 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

| International application No. |
|---|
| **PCT/JP2024/027247** |

| Patent document cited in search report | | | Publication date (day/month/year) | Patent family member(s) | | | Publication date (day/month/year) |
|---|---|---|---|---|---|---|---|
| EP | 2887050 | A1 | 24 June 2015 | WO | 2015/097089 | A1 | |
| US | 2010/0145199 | A1 | 10 June 2010 | WO | 2007/099336 | A1 | |
| JP | 2005-534899 | A | 17 November 2005 | US | 2005/0074745 | A1 | |
| | | | | paragraphs [0031]-[0034] | | | |
| | | | | US | 2009/0192721 | A1 | |
| | | | | WO | 2003/107270 | A2 | |
| | | | | EP | 1540560 | A2 | |
| | | | | CA | 2487836 | A1 | |
| | | | | CN | 1701334 | A | |
| | | | | KR | 10-2005-0012281 | A | |
| | | | | AU | 2003232406 | A | |
| CN | 111024624 | A | 17 April 2020 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 58118948 A

- JP 6959614 B

**Non-patent literature cited in the description**

- **SHUNICHIRO OGURA et al.** Dormant cancer cell-targeted photodynamic therapy using 5-aminolevulinic acid. *The Journal of Japan Society for Laser Surgery and Medicine*, 2023, vol. 43 (4), 238-248
- **A. WAX**. Cellular organization and substructure measured using angle-resolved low-coherence interferometry. *Biophysical journal*, 2002, vol. 82 (4), 2256-2264

- **V. BACKMAN**. Polarized light scattering spectroscopy for quantitative measurement of epithelial cellular structures in situ. *IEEE Journal of Selected Topics in Quantum Electronics*, 1999, vol. 5 (4), 1019-1026
- **M. XU**. Unified Mie and fractal scattering by cells and experimental study on application in optical characterization of cellular and subcellular structures. *Journal of biomedical optics*, 2008, vol. 13 (2), 024015-024015